# EUROPÄISCHE PATENTANMELDUNG

(11) **EP 0 585 702 A1**
(43) Veröffentlichungstag der Anmeldung: **09.03.1994**
(21) Anmeldenummer: 93113059.5
(22) Anmeldetag: 14.08.1993
(51) Int. Cl.: G01N 33/18, C02F 3/00, G05D 21/02

(54) **Anordnung zur Regelung der Bioaktivität von biologischen Systemen**

(30) Priorität: 04.09.1992 DE 4229550
(71) Anmelder: GRUNDIG E.M.V. Elektro-Mechanische Versuchsanstalt Max Grundig GmbH & Co. KG, D-90762 Fürth (DE)
(72) Erfinder: Hallas, Ernst, Dr., GRUNDIG E.M.V., D-90748 Fürth (DE)

(57) **Zusammenfassung**

Die Erfindung betrifft ein Verfahren und eine Anordnung zur Regelung der Bioaktivität von biologischen Systemen, insbesondere in biologischen Abwasserreinigungssystemen, bei denen zur Aufrechterhaltung der Bioaktivität Sauerstoff zugeführt wird.
Um die biologische Abwasserreinigung effektiv zu gestalten ist es bekannt, die Sauerstoffverbrauchsrate zu bestimmen und in Abhängigkeit dieser Sauerstoffverbrauchsrate durch definierte Sauerstoffzufuhr die biologischen Prozesse der Abwasserreinigung zu steuern. Dies ist notwendig und zweckmäßig, da in Abwasserreinigungsanlagen sowohl über den Tag als auch über die Woche verteilt Schwankungen in der biologischen Belastung des Abwassers auftreten. Aufgabe der Erfindung ist es deshalb, eine Vorrichtung anzugeben, die eine zuverlässige Bestimmung und Regelung der Bioaktivität zuläßt.

Erfindungsgemäß wird diese Aufgabe gelöst, indem das in einem Belebtschlammbecken freigesetzte Gas über der Oberfläche des Belebtschlammbeckens in einem oder mehreren Bereichen von der Umgebungsatmosphäre abgeschirmt wird. Die Konzentration des Gases innerhalb der Abschirmung wird mittels einer Meßsonde bestimmt.

## Beschreibung

Die Erfindung betrifft eine Anordnung zur Regelung der Bioaktivität von biologischen Systemen, insbesondere in biologischen Abwasserreinigungssystemen, bei denen zur Aufrechterhaltung der Bioaktivität Sauerstoff zugeführt wird.

In Abwasserreinigungsanlagen werden zunächst auf mechanischem Weg die ungelösten Stoffe im Abwasser abgeschieden. Im weiteren werden im Rahmen einer biologischen Reinigung biologisch abbaubare Wasserinhaltsstoffe abgebaut. Voraussetzung für die biologische Abbaubarkeit ist im wesentlichen das Vorhandensein von kohlenstoffhaltigem organischen Material und vor allem von Sauerstoff.
Wegen seiner großen Anpassungsfähigkeit an unterschiedliche Belastungen wird neuerdings überwiegend das Belebtschlammverfahren oder Belebungsverfahren mit unterschiedlichen Mikroorganismen angewandt. Hier schwimmen Bakterienkolonien frei in einem mit dem zu reinigenden Abwasser durchströmten bzw. gefüllten Belebungsbecken. Der zum Abbau der Schadstoffe benötigte Sauerstoff wird beispielsweise mit Belüftern in das Belebungsbecken eingebracht.
Die Belüftung kann sowohl von der Oberfläche her durch Verwirbelung oder aus der Tiefe durch Luft- bzw. Sauerstoffeinblasen erfolgen.

Um die biologische Reinigung effektiver zu gestalten, ist es bekannt, die Sauerstoffverbrauchsrate über die Größe des Sauerstoffüberschusses in Form von gelöstem Sauerstoff mit Partialdrucksonden zu bestimmen und in Abhängigkeit dieser Sauerstoffverbrauchsrate durch definierte Sauerstoffzufuhr die biologischen Prozesse der Abwasserreinigung zu steuern. Dies ist notwendig und zweckmäßig, da in Abwasserreinigungsanlagen sowohl über den Tag als auch über die Woche verteilt Schwankungen in der biologischen Belastung des Abwassers auftreten. Diese Schwankungen werden durch die unterschiedlich starken Abwassereinläufe aus der Industrie und den privaten Haushalten verursacht. So beinhalten die Abwasser beispielsweise während der Nachtstunden oder an Wochenenden nur ein Viertel oder weniger biologisch abbaubare Stoffe als bei Lastspitzen.

Nachteil dieser Steuerung ist, daß man die Anlage entsprechend dem chemischen Sauerstoffbedarf (CSB) oder dem biologischen Sauerstoffbedarf (BSB) mit im allgemeinen hohem Luft- bzw. Sauerstoffüberschuß betreibt, um stets einen sicher meßbaren Sauerstoffüberschußpartialdruck zu erhalten.
Der hohe Sauerstoffüberschuß kann nur mit erheblichem Energieverbrauch für die Belüftung erreicht werden.

Weiterhin gibt die Sauerstoffüberschußpartialdruckmessung nur eine indirekte Sauerstoffzehrungsaussage an, die vergleichsweise ungenau ist.

In der europäischen Offenlegungsschrift EP 0 414 182 A1 wird deshalb eine Vorrichtung zur Bestimmung der biochemischen Sauerstoffverbrauchsrate angegeben, die ein Reaktionsgefäß für die Aufnahme von Mikroorganismen und Substratflüssigkeit umfaßt. Das Reaktionsgefäß ist über einen CO₂-Absorber mit einem Sauerstofferzeuger und einem Druckindikator verbunden. Die Vorrichtung enthält weiterhin ein Registriergerät für die erzeugten Sauerstoffmengen. Zusätzlich ist im Reaktionsgefäß eine Sauerstoffmeßsonde angebracht, die mit einem Meß- und Steuerrechner verbunden ist, der über die zeitliche Gelöstsauerstoffänderung korrigierte Sauerstoffverbrauchsdaten angibt.

Der Nachteil dieser Vorrichtung wird darin gesehen, daß im Reaktionsgefäß lediglich eine Probe, angegeben ist ein typisches Volumen des Reaktionsgefäßes von 300 ml, ausgewertet wird, die nicht zwangsläufig die Situation im Belebtschlammbecken wiedergibt.

Aufgabe der Erfindung ist es deshalb, eine Vorrichtung anzugeben, die eine zuverlässige Bestimmung und Regelung der Bioaktivität zuläßt.

Erfindungsgemäß wird diese Aufgabe gelöst, indem das in einem Belebtschlammbecken freigesetzte Gas über der Oberfläche des Belebtschlammbeckens in einem oder mehreren Bereichen von der Umgebungsatmosphäre abgeschirmt wird. Die Konzentration des Gases innerhalb der Abschirmung wird mittels einer Meßsonde bestimmt. Im folgenden wird aus dem Meßergebnis eine Beurteilungsgröße für die Sauerstoffzehrung gewonnen und durch den Vergleich mit einer Sollgröße wird eine Regelgröße bestimmt. Mit dieser Regelgröße wird ein Stellglied angesteuert, mit dem eine bedarfsgerechte Zuführung des Regelgases in das Belebtschlammbecken erreicht wird.

Ein Vorteil der erfindungsgemäßen Lösung liegt darin, daß die Abgase an der Oberfläche an der oder den Meßstellen von der Umgebungsatmosphäre abgeschirmt sind, und somit eine Vermischung ausgeschlossen ist. Es wird also das Abgas ohne verfälschende Einflüsse gemessen, wobei gleichzeitig nicht nur die Bioaktivität einer kleinen Probe bestimmt wird, sondern die aktuelle Aktivität an beliebigen Meßpunkten eines Beckens.

Eine vorteilhafte Ausgestaltung der Erfindung sieht vor, daß mindestens einer der Parameter Sauerstoff oder CO₂ bestimmt wird. Insbesondere bei der gleichzeitigen Bestimmung beider Parameter zur Berechnung eines Zehrungsparameters ergeben sich Vorteile, da hierbei die Luftzufuhr so geregelt werden kann, daß nur der O₂-Gehalt, nicht aber der CO₂-Gehalt absinkt, wodurch sich die Möglichkeit einer erheblichen Energieeinsparung gegenüber bekannten Sauerstoffüberschußpartialdruckmessungen, mit dem dafür notwendigen hohen Luftüberschuß im Belebungsbecken, ergibt.

Eine weitere vorteilhafte Ausgestaltung der Erfindung ergibt sich durch die Messung der leicht flüchtigen Kohlenwasserstoffe. Durch diese Messung kann rechtzeitig und wirksam eine Änderung der Bioaktivität durch HC-Vergiftung angezeigt und zur Alarmierung verwendet werden.

Als vorteilhaft erweist sich auch eine lokale Aufheizung im Bereich der abgeschirmten Meßstelle bzw. der abgeschirmten Meßstellen. Durch diese Aufheizung werden die Gasungserscheinungen verstärkt, so daß eine genauere Messung erreicht werden kann. Dies bringt insbesondere Vorteile bei der Messung von schwerer ausgasenden Komponenten.

Weiterhin hat es sich als vorteilhaft erwiesen, die Messungen intermittierend durchzuführen. Bei der intermittierenden Messung wird innerhalb einer vorgebbaren Zykluszeit, beispielsweise 15 Minuten, innerhalb einer weiteren vorgebbaren Zeit, beispielsweise 5 Minuten, eine Messung durchgeführt. Die verbleibende Zeit kann für automatische Abgleiche, beispielsweise Frischluftkalibrierung oder Selbsttest verwendet werden, oder als Pause, wodurch sich Einsparungen in Form von Verschleißminderung der Meßgeräte ergeben.

Für die bereichsweise Abdeckung der Oberfläche eines Belebtschlammbeckens hat sich eine Abdeckhaube als günstig erwiesen. Diese Abdeckhaube wird mit Hilfe von Schwimmkörpern auf der Oberfläche gehalten, wobei Befestigungseinrichtungen vorgesehen sind, die die Abdeckhaube in einer bestimmten Position halten. Die sich unter der Abdeckung bildenden Gase werden mittels einer flexiblen Gasabführungsleitung einer Meßeinrichtung zur Bestimmung der Konzentration zugeführt. In einer der Meßeinrichtung zugeordneten Auswerteeinrichtung werden in Abhängigkeit der Meßergebnisse Stellwerte ermittelt, mit denen ein Stellglied zur Zuführung des oder der Reaktionsgase angesteuert wird. Die Meßwerte können auch einer Warte zur Kontrolle und Auswertung zugeführt werden.

In vorteilhafter Weise werden die Schwimmkörper an der Abdeckhaube derart angebracht, daß diese eine vorgebbare Eintauchtiefe in die Oberfläche des biologischen Systems, also des Belebtschlammbeckens, erreicht.
Durch das Eintauchen der Abdeckhaube wird sichergestellt, daß auch bei Turbulenzen an der Oberfläche keine Verfälschungen des Meßergebnisses durch die Umgebungsatmosphäre auftreten.

Eine stabilisierende Wirkung auf die Vorrichtung wird durch das Anbringen einer stabilisierenden Masse unterhalb des Schwerpunktes der Abdeckung erreicht. Diese Masse kann an der Abdeckhaube oder an den Schwimmkörpern befestigt sein.

Als Stellglieder für die Zuführung des Reaktionsgases werden in vorteilhafter Weise die bereits vorhandenen Belüftungspumpen verwendet. Diese Belüftungspumpen können durch die Regelung der Drehzahl die Zuführung insbesondere von Sauerstoff in der gewünschten Weise beeinflussen.

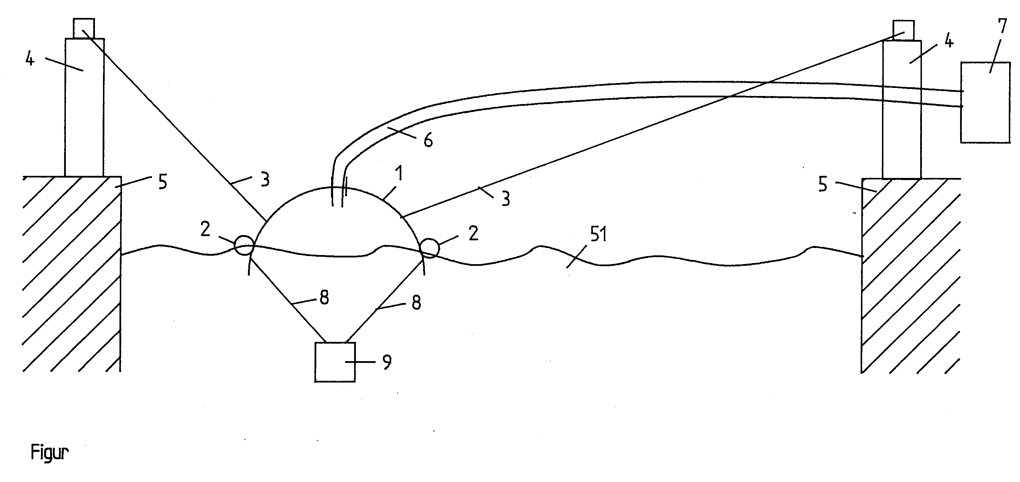

Im weiteren wird die Erfindung an Hand der Figur näher erläutert.
Die Figur zeigt den Querschnitt einer möglichen Anwendung der Erfindung in einem Belebtschlammbecken einer Klärwerksanlage.
Gemäß der Figur ist auf der Belebtschlammoberfläche 51 des Belebtschlammbeckens 5 eine Abdeckhaube 1 schwimmend angeordnet. An der Abdeckhaube 1 sind Schwimmkörper 2 angebracht, die die Abdeckhaube 1 über der Oberfläche 51 halten. Die Schwimmkörper sind in einer Höhe angebracht, bei der die Abdeckhaube 1 im unteren Teil in die Belebtschlammoberfläche eintaucht. Die Eintauchtiefe ist so gewählt, daß auch bei Turbulenzen auf der Oberfläche sichergestellt ist, daß die Umgebungsatmosphäre sicher abgeschirmt ist, d.h. daß sich die Enden der Abdeckhaube auch bei Turbulenzen an der Oberfläche 51 immer unterhalb der Oberfläche befinden.
Damit die Abdeckhaube 1 in ihrer Lage weitgehend stabil ist, ist sie mit mindestens zwei flexiblen Befestigungsbändern 3 verbunden, die am Beckenrand 5 an Halterungen 4 befestigt sind.
Zur zusätzlichen Stabilisierung der Abdeckhaube 1 ist an mindestens einer Halterung 8 eine Masse 9 derart angebracht, daß der Schwerpunkt der Gesamtanordnung nach unten verlagert wird. Neben dem Effekt der Verlagerung des Schwerpunktes spielt hierbei für die Stabilisierung auch die Trägheit der Masse 9 eine Rolle, da durch diese Trägheit Krafteinwirkungen auf die Abdeckhaube 1 an der Oberfläche zum Teil ausgeglichen werden.

Die stabilisierende Masse 9 kann zugleich ein Heizelement beinhalten, mit dem eine lokale Aufheizung zur Verstärkung der Gasungserscheinungen, und somit zur Verbesserung der Auswertbarkeit, vorgenommen werden kann.

Durch die beschriebene Vorrichtung werden die durch biologische Aktivität im Belebtschlammbecken freigesetzten Gase von der Umgebungsatmosphäre abgeschirmt. Dadurch wird eine unverfälschte Messung der freigesetzten Gase ermöglicht. Die an der Belebtschlammoberfläche 51 freigesetzten Gase werden mittels eines flexiblen Abgasschlauches 6 an die Meß- und Auswertevorrichtung 7 geführt. In der Meß- und Auswerteeinrichtung 7 werden die interessierenden Abgasbestandteile bestimmt und Stellwerte für Stellglieder, die in der Figur nicht dargestellt sind, errechnet. Als Stellglieder können insbesondere Belüftungspumpen oder Rührwerke, die im Belebtschlammverfahren für Abwasserreinigung ohnehin vorhanden sind, zur Regelung der Bioaktivität durch Zufuhr von Sauerstoff verwendet werden.

In der Figur wurde aus Gründen der Übersichtlichkeit nur eine Abdeckhaube dargestellt. Im realen Einsatz in Belebtschlammbecken von Klärwerksanlagen ist es auf Grund der großen Oberflächenabmessungen vorteilhaft mehrere Abdeckhauben anzubringen. Dies gilt insbesondere für Belebtschlammbecken, bei denen eine ungleiche Bioaktivität bezogen auf die Belebtschlammoberfläche zu erwarten ist. Bei dem Einsatz mehrerer Abdeckhauben kann eine Meß- und Auswerteeinrichtung 7 vorgesehen sein, an die die Abgasschläuche 6 der einzelnen Abdeckhauben 1 geführt werden. Es ist aber in speziellen Anwendungsfällen auch sinnvoll, für jede Abdeckhaube eine gesonderte Meß- und Auswerteeinrichtung vorzusehen.

## Patentansprüche

1. Vorrichtung zur Regelung der Bioaktivität von biologischen Systemen, insbesondere in biologischen Abwasserreinigungssystemen, bei denen zur Aufrechterhaltung der Bioaktivität Sauerstoff zugeführt wird und bei denen in Abhängigkeit der Bioaktivität Gas freigesetzt wird, **dadurch gekennzeichnet**, daß
- das freigesetzte Gas über der Oberfläche von der Umgebungsatmosphäre in ein oder mehreren Bereichen abgeschirmt wird,
- die Konzentration des Gases innerhalb der Abschirmung mittels einer Meßsonde bestimmt wird,
- aus dem Meßergebnis eine Beurteilungsgröße für die Sauerstoffzehrung bestimmt wird und durch Vergleich mit einer Sollgröße eine Regelgröße bestimmt wird, die mindestens ein entsprechendes Stellglied derart ansteuert, daß eine bedarfsgerechte Zuführung des Reaktionsgases erfolgt.

2. Vorrichtung nach Anspruch 1, **dadurch gekennzeichnet**, daß
mit der Meßsonde mindestens einer der Parameter O₂ oder CO₂ bestimmt wird.

3. Vorrichtung nach Anspruch 1 oder 2, **dadurch gekennzeichnet**, daß aus der gleichzeitigen Bestimmung des O₂- und des CO₂-Gehaltes ein Zehrungsparameter bestimmt wird, und in Abhängigkeit des Zehrungsparameters die Luftzufuhr bei konstantem CO₂ verändert wird.

4. Vorrichtung nach einem oder mehreren der Ansprüche 1 bis 3, **dadurch gekennzeichnet**, daß zusätzlich der Anteil von leicht flüchtigen Kohlenwasserstoffen bestimmt wird und das Ergebnis der Bestimmung als Kontrollparameter verwendet wird, der eine Veränderung der Bioaktivität durch HC-Vergiftung angibt.

5. Vorrichtung nach einem oder mehreren der Ansprüche 1 bis 4, **dadurch gekennzeichnet**, daß zur Verstärkung der Gasungserscheinungen eine lokale Aufheizung im Bereich einer Meßsonde vorgenommen wird.

6. Vorrichtung nach einem oder mehreren der Ansprüche 1 bis 5, **dadurch gekennzeichnet**, daß die Bestimmungen der Parameter intermittierend erfolgt.

7. Vorrichtung nach einem oder mehreren der Ansprüche 1 bis 6, **gekennzeichnet durch**
- mindestens eine Abdeckhaube zur bereichsweisen Abschirmung der Oberfläche (51) eines biologischen Systems,
- an der Abdeckhaube (1) angebrachte Schwimmkörper (2), die die Abdeckhaube (1) tragen,
- Befestigungseinrichtungen (3), die die Abdeckhaube (1) in einer bestimmbaren Position halten,
- eine flexible Gasabführungsleitung (6) zur Zuführung des zu bestimmenden Gases an eine Meß- und Auswerteeinrichtung (7), die die Konzentration des Gases bestimmt und die in Abhängigkeit des Meßergebnisses Stellwerte ermittelt, und
- mindestens ein Stellglied, das die Zufuhr von Reaktionsgas steuert.

8. Vorrichtung nach Anspruch 7, **dadurch gekennzeichnet**, daß die Schwimmkörper (2) an der Abdeckhaube (1) derart angebracht sind, daß die Abdeckhaube (1) eine vorgebbare Eintauchtiefe in die Oberfläche (51) des biologischen Systems hat.

9. Vorrichtung nach Anspruch 7 oder 8, **dadurch gekennzeichnet**, daß an der Abdeckhaube (1) oder an den Schwimmkörpern (2) eine stabilisierende Masse (9) unterhalb des Schwerpunktes der Abdeckhaube (1) angebracht ist.

10. Vorrichtung nach einem oder mehreren der Ansprüche 7 bis 9, **dadurch gekennzeichnet**, daß die Stellglieder Belüftungspumpen sind.
